# DEMANDE DE BREVET EUROPEEN

(11) **EP 1 384 490 A1**
(43) Date de publication de la demande: **28.01.2004**
(21) Numéro de dépôt: 02405639.2
(22) Date de dépôt: 22.07.2002
(51) Int. Cl.: A61M 5/142

(54) **Pompe programmable pour l'injection de médicaments**

(71) Demandeur: Precimedix S.A., 2000 Neuchâtel (CH)
(72) Inventeur: Möri, Peter, 3272 Walperswill (CH); Wüthrich, Heinz, 3048 Wordblaufen (CH); Stulz, Beat, 1700 Fribourg (CH); Clement, Claude, 1333 Lussy-sur-Morges (CH)
(74) Mandataire: Gresset, Jean

(57) **Abrégé**

L'invention concerne une pompe programmable pour l'injection de médicaments, comportant deux parties accouplables de manière amovible, soit :
- un module cassette (12) contenant un liquide à injecter et comprenant une mémoire électronique dans laquelle ont été préalablement enregistrées des données relatives au programme d'injection prescrit, et
- un module pompe (10) possédant des moyens d'actionnement qui, lors de l'accouplement, répondent directement aux données contenues dans ladite mémoire pour faire passer le liquide du module cassette (12) vers l'extérieur selon le programme désiré.

Les modules pompe (10) et cassette (12) sont accouplés au moyen d'une articulation (36) à baïonnette. Le module pompe (10) comporte une mémoire dans laquelle ont été préalablement enregistrées des données de sécurité relatives au traitement. Les moyens d'actionnement comportent un microprocesseur programmé pour vérifier la compatibilité des données contenues dans les deux mémoires.

## Description

La présente invention se rapporte au domaine des pompes miniaturisées pour l'injection de médicaments selon un programme prescrit. Elle concerne, plus particulièrement, une pompe médicale programmable formée d'un module pompe et d'un module cassette contenant le médicament, qui s'accouplent de manière amovible.

Les pompes miniaturisées à usage médical sont connues depuis plusieurs années. Légères et de faibles dimensions, elles sont portées par le patient de façon discrète et sans inconfort et permettent de lui administrer, par voie sous-cutanée ou intraveineuse, en continu ou selon un programme déterminé, des quantités contrôlées de solutions médicamenteuses, sans qu'il soit, pour autant, alité et relié à un appareil encombrant, coûteux et bruyant.

De telles pompes sont, souvent, de type péristaltique rotatif. Leur principe consiste à disposer d'un tuyau en matière plastique déformable relié à un réservoir contenant la solution de médicament et à l'écraser localement contre une pièce d'appui de forme arrondie au moyen de galets presseurs montés sur un rotor entraîné par un moteur agissant par l'intermédiaire d'un train d'engrenages. Le liquide est ainsi aspiré du réservoir et poussé vers la sortie pour être injecté dans le corps du patient.

Le document FR 2 753 235, par exemple, décrit des pompes à cassette de ce type.

Lors de la conception de ces pompes, il est particulièrement important de se préoccuper de leur facilité de programmation. Destinées surtout à être utilisées en milieu non hospitalier, elles doivent, en effet, pouvoir être mises en service, de manière sûre, par tout personnel soignant, voire par le patient lui-même, sans qu'il ait reçu une formation spéciale ou doive lire un long et compliqué mode d'emploi. Or, ces exigences de convivialité et de sécurité sont rarement satisfaites par les pompes actuellement sur le marché. Le risque est, alors, qu'elles restent un équipement d'hôpital réservé à quelques infirmières, ce qui leur fait perdre une grande partie de leur intérêt.

Le document PCT/CH 02/00190 propose une pompe programmable à cassette immédiatement utilisable par toute personne, sans nécessiter aucune formation ou connaissance particulière. Elle comporte deux modules accouplables de manière amovible, soit :
- un module cassette contenant le liquide à injecter et doté d'une mémoire électronique dans laquelle ont été préalablement enregistrées des données relatives au programme d'injection prescrit, et
- un module pompe possédant des moyens d'actionnement commandés par un microprocesseur qui, lors de l'accouplement, répondent directement aux données contenues dans la mémoire pour faire passer le liquide du module cassette vers l'extérieur selon le programme désiré.

Malgré l'intérêt qu'il présente, ce dispositif n'est pas sans inconvénient. En effet, le module cassette s'emboîte et coulisse dans le module pompe à la façon d'un tiroir. La rigidité de l'ensemble n'est donc pas satisfaisante et l'accouplement mécanique des deux modules ne se fait pas de manière optimale. De plus, le positionnement relatif des pistes conductrices qui, lors de l'accouplement, doivent se mettre en communication pour interconnecter la mémoire et le microprocesseur, n'est pas suffisamment précis.

La présente invention se propose donc de pallier ces inconvénients grâce à un système d'assemblage des modules cassette et pompe particulièrement rigide et assurant un accouplement très facile et précis des modules aussi bien du point de vue mécanique que du point de vue électrique.

L'invention a aussi pour but de fournir une pompe programmable qui garantit une sécurité optimale en évitant toute erreur de programmation.

De façon plus précise, l'invention concerne une pompe programmable pour l'injection de médicaments, comportant deux parties accouplables de manière amovible, telles que définies ci-dessus, mais qui est caractérisée en ce que les modules pompe et cassette sont accouplés au moyen d'une articulation.

De façon particulièrement avantageuse, l'articulation entre les deux modules est de type à baïonnette et comporte une partie mâle appartenant à l'un des modules et une partie femelle appartenant à l'autre module. La partie mâle est de forme cylindrique et possède, à sa base, une gorge horizontale formée sur tout son pourtour et deux gorges verticales communiquant avec la gorge horizontale. La partie femelle est constituée d'un cylindre creux dont le diamètre intérieur est légèrement supérieur à celui de la partie mâle et qui possède deux ergots intérieurs, disposés et dimensionnés de manière à pouvoir s'insérer et coulisser dans les gorges de la partie mâle.

La pompe selon l'invention comporte encore, de préférence, tout ou partie des caractéristiques suivantes :
- un verrou sert à solidariser le module pompe et la cassette lorsqu'ils sont accouplés ;
- le module pompe est de type péristaltique rotatif à galets, alors que le module cassette comporte une poche destinée à contenir le liquide à injecter, un tuyau souple reliant la poche à l'extérieur et une contre-pièce sur laquelle les galets viennent tour à tour écraser le tuyau et pousser ainsi vers l'extérieur le liquide contenu dans la poche ;
- la mémoire du module cassette est une carte SIM et le module pompe comporte un connecteur relié aux moyens d'actionnement et positionné de manière à ce que, lors de l'accouplement des deux modules, ses plages de contact viennent précisément au contact des plages de la carte SlM ;
- la carte SIM est disposée, de manière amovible, sur la contre-pièce du module péristaltique;
- le module pompe comporte une mémoire dans laquelle ont été préalablement enregistrées des données de sécurité relatives au traitement ; et
- les moyens d'actionnement comportent un microprocesseur programmé pour vérifier la compatibilité des données contenues dans les deux mémoires.

L'invention concerne également une pompe programmable pour l'injection de médicaments, du type comportant deux parties accouplables de manière amovible, telles que définies précédemment. Elle est caractérisée en ce que le module pompe comporté une mémoire dans laquelle ont été préalablement enregistrées des données de sécurité relatives au traitement et en ce que les moyens d'actionnement comportent un microprocesseur programmé pour vérifier la compatibilité des données contenues dans les deux mémoires.

L'invention sera mieux comprise à la lecture de la description qui suit, faite en regard du dessin annexé, sur lequel ;
- la figure 1 représente, de face en a et de dos en b, une pompe programmable selon l'invention, les modules pompe et cassette étant assemblés;
- la figure 2 est une vue du module pompe, éclaté en a et assemblé en b, la coque supérieure étant enlevée;
- la figure 3 est une vue du module cassette, en éclaté en a et assemblé en b;
- la figure 4 est une vue agrandie, de l'avant en a et de l'arrière en b, de la contre-pièce du module cassette ; enfin,
- la figure 5 sert à illustrer la manière dont les modules pompe et cassette sont articulés.

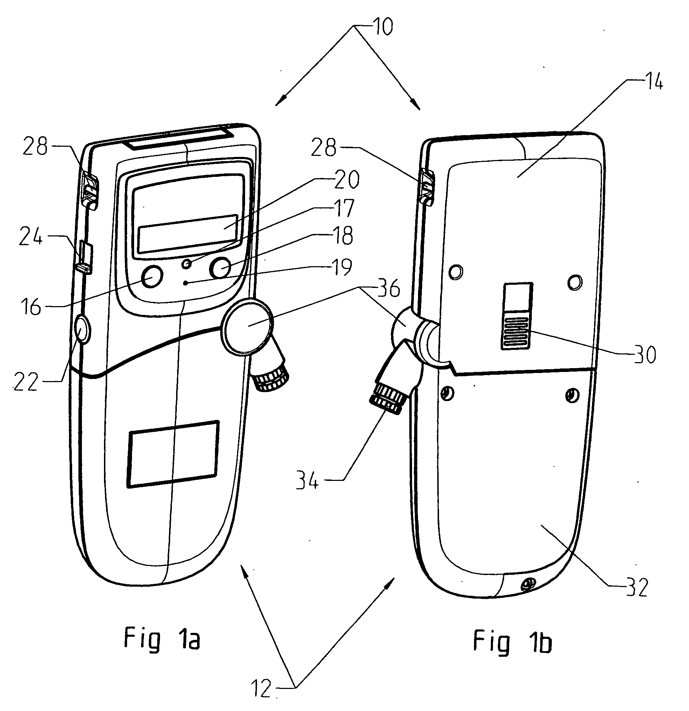

Sur la figure 1, on a représenté une pompe à module cassette programmable selon l'invention, formée d'un module pompe 10 et d'un module cassette 12 s'accouplant au module pompe de manière amovible et articulée.

Le module pompe 10 comporte un boîtier en plastique rigide 14 ouvert sur son côté en regard du module cassette 12. Sa face avant possède un bouton START/STOP 16 servant à commander le démarrage et l'arrêt de la pompe, une diode électroluminescente (LED) 17 servant d'alarme visuelle et d'indicateur de marche, un bouton BOLUS 18 servant à déclencher l'administration de doses additionnelles de médicament, un orifice 19 pour un microphone et un affichage LCD 20.

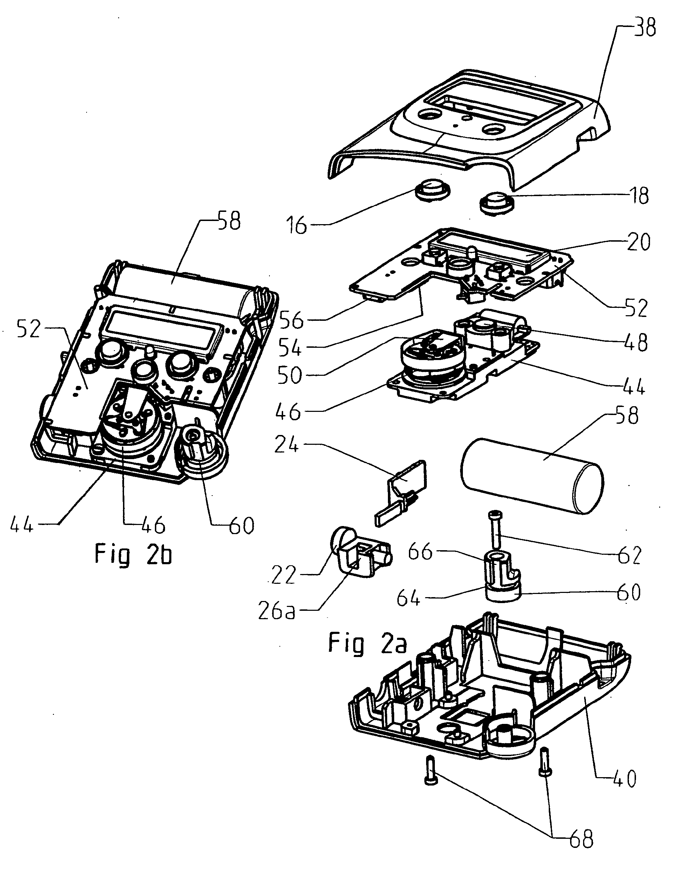

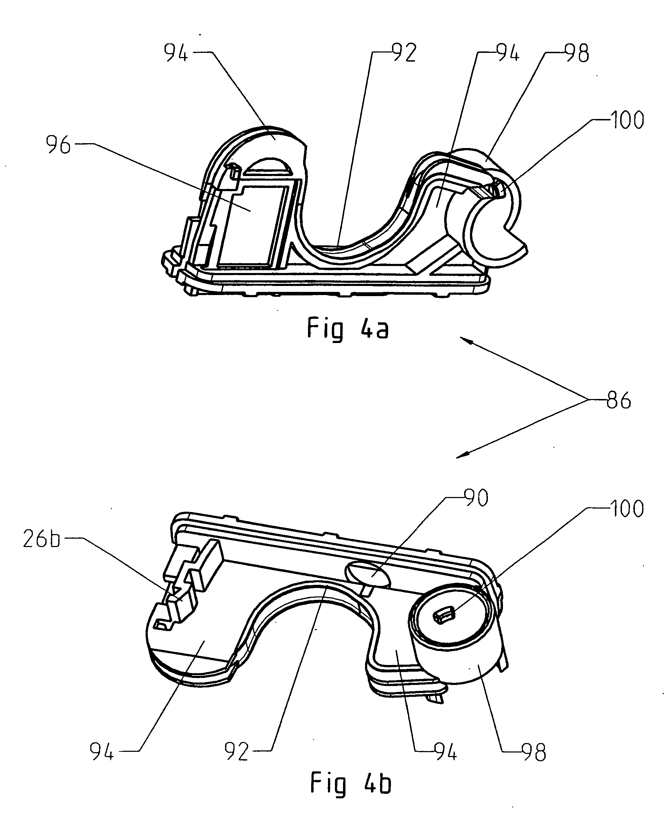

L'une des faces latérales du boîtier 14 porte un bouton 22 et une tirette 24, montés mobiles et servant tous deux à actionner un verrou interne 26 visible sur les figures 2 et 4 et destiné à solidariser le module pompe et la cassette lorsqu'ils sont assemblés. Sur chacune de ses faces latérales, du côté opposé au module cassette, le boîtier 14 comporte, en outre, deux barrettes 28 autour desquelles on peut fixer un cordon pour accrocher la pompe, par exemple autour du cou du porteur.

La face arrière du boîtier 14 présente un clapet de purge 30 qui permet d'actionner la pompe de l'extérieur à vitesse élevée afin d'évacuer l'air subsistant dans les tuyaux. Le système utilisé est celui décrit dans le document FR précité.

Le module cassette 12 comporte aussi un boîtier en plastique rigide 32 ouvert sur son côté en regard du module pompe 10. Il est doté d'un raccord 34, du type Luer, bien connu de l'homme de métier, destiné à connecter un tuyau souple (non représenté sur la figure) qui se termine par une aiguille d'injection pour administrer au patient le médicament contenu dans la cassette.

Les boîtiers 14 et 32 sont liés l'un à l'autre par une articulation à baïonnette 36 formée à un de leurs angles en regard. Cette articulation sera décrite plus loin en détail.

Comme le montrent les figures 2a et 2b, le boîtier 14 du module pompe 10 est formé d'une coque supérieure 38 et d'une coque inférieure 40. Outre l'orifice 19, des trous ont été ménagés dans la coque 38 pour laisser passer les boutons 16 et 18, la diode 17 ainsi que l'affichage 20. La coque inférieure 40 est structurée de manière à former des logements pour maintenir en place, coincées entre elle et la coque supérieure 38, toutes les pièces contenues dans le boîtier 14. Cette technique est bien connue de l'homme de métier et la structure d'appui ne sera pas décrite plus en détail.

Une platine métallique 44 est vissée sur la coque inférieure 40. Elle sert de base à une tête de pompe 46 entraînée par un moteur pas à pas 48 au travers d'un train d'engrenage 50. La tête 46, qui apparaît à proximité du côté ouvert du boîtier 14, est de type péristaltique rotative à trois galets presseurs. Une description détaillée de cette tête de pompe est fournie dans le document FR susmentionné.

La coque inférieure 40 reçoit un circuit imprimé rigide 52 découpé en forme de L de manière à libérer un espace suffisant pour la tête de pompe 46. Ce circuit sert de base à un microprocesseur et une mémoire, tous deux non visibles sur la figure, car disposés sur la face inférieure du circuit. Le circuit imprimé 52 porte aussi les boutons 16 et 18 et l'affichage 20. Son réseau de pistes conductrices assure l'interconnexion de ces éléments entre eux ainsi qu'avec le moteur 48 et deux connecteurs 54 et 56 disposés à proximité du côté ouvert du boîtier 14, destinés à assurer la liaison électrique du module pompe 10 respectivement avec une interface de programmation et avec le module cassette 12. Le circuit imprimé 52 est également relié à un accumulateur 58 disposé dans le boîtier 14 du côté opposé au module cassette 12.

La coque inférieure 40 possède, au niveau de son côté ouvert, à l'angle opposé à celui où se trouvent les deux connecteurs 54 et 56, la partie mâle 60 de l'articulation à baïonnette 36, fixée par une vis 62. Cette partie mâle est de forme cylindrique et possède, à sa base, une gorge horizontale 64 formée sur son pourtour dans laquelle débouchent deux gorges verticales 66.

La partie 26a du verrou est maintenue dans la coque inférieure 40, du côté des deux connecteurs 54 et 56. Elle est constituée d'une rainure verticale en forme de L ménagée dans le corps du bouton 22. La tirette 24 est structurée et disposée de manière à ce que, lorsqu'elle est dans sa position de repos, elle bloque toute translation du bouton 22. Ainsi, il est nécessaire d'actionner la tirette 24 pour pouvoir déplacer le bouton 22 qui permet de désolidariser les deux modules .

Enfin, deux vis 68 assurent l'assemblage des deux coques 38 et 40 de manière à maintenir en place les différentes pièces abritées dans le boîtier 14.

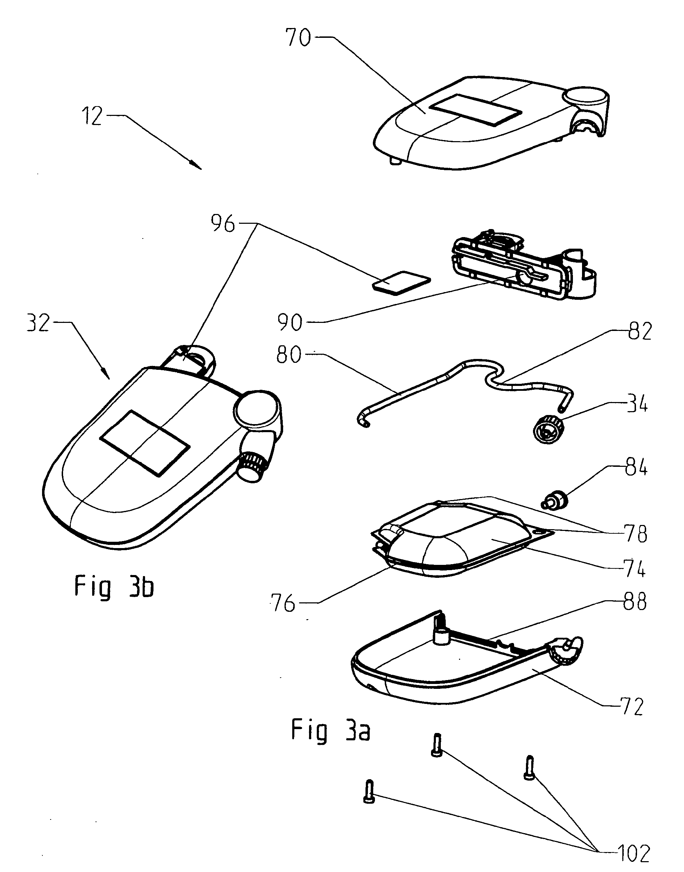

Le module cassette 12 et ses constituants vont maintenant être décrits en référence aux figures 3 et 4. Son boîtier 32 comporte une coque supérieure 70 et une coque inférieure 72 qui sont structurées de manière à fournir des appuis pour maintenir en place, coincées entre elles, toutes les pièces logées à l'intérieur.

Une poche réservoir de médicament 74 est disposée dans le boîtier 32. Elle est en plastique souple inextensible choisi pour sa compatibilité avec le médicament qu'elle doit recevoir et présente, sur son pourtour, un rebord plat 76 formant deux coins 78 percés d'un trou au niveau de ses extrémités en regard du module pompe 10. La poche 74 possède, à sa base, c'est à dire à l'opposé du module pompe, un premier orifice dans lequel est insérée l'extrémité d'un tube connecteur 80 la reliant hermétiquement au raccord 34 par l'intermédiaire d'un tuyau souple 82. Du côté du module pompe, la poche 74 est dotée d'un deuxième orifice obturé par un septum 84. C'est à travers lui que le réservoir est rempli de médicament au moyen d'une aiguille et d'une seringue.

Le tuyau souple 80 est appliqué sur une contre-pièce 86, montrée en détail sur la figure 4, qui occupe toute la largeur du boîtier 32 du côté du module pompe 10 et est positionnée par une rainure 88 de la coque inférieure 72. Cette pièce est percée d'un trou 90 permettant d'accéder au septum 84 et comporte, en son milieu, une partie concave arrondie 92, en forme de U, dont le rayon est légèrement supérieur à celui du cercle que parcourt la face externe des galets de la tête de pompe 46.

La partie concave 92 de la contre-pièce 86 définit une zone d'appui sur laquelle, une fois les deux modules assemblés, lors de la rotation du rotor de la tête de pompe 46, les trois galets presseurs de celle-ci viennent tour à tour écraser le tuyau souple 82 et pousser ainsi vers l'extérieur, par un mouvement péristaltique, le liquide contenu dans la poche 74.

La partie concave 92 de la contre-pièce 86 est au milieu de deux parties convexes 94 qui possèdent une gorge dans laquelle prend place le tuyau souple 82, ainsi guidé et maintenu en place de manière très précise.

L'une des parties convexes 94 de la pièce 86 sert de support à un circuit intégré mémoire 96, avantageusement constitué d'une carte de type SIM (Subscriber Information Memory) positionnée de manière à ce que, lorsque les deux modules de la pompe sont assemblés, ses plages de contact viennent précisément au contact des plages du connecteur 56 appartenant au module pompe 10.

Sur la partie convexe 94 qui porte la carte SIM 96, mais sur sa face opposée, la contre-pièce 86 reçoit encore la partie 26b du verrou servant à solidariser les deux modules lorsque la pompe est en fonctionnement. Cette partie est constituée d'un crochet destiné à coopérer avec la rainure ménagée dans le bouton 22.

La contre-pièce 86 possède, à son extrémité opposée à celle qui porte la carte SIM 96, la partie femelle 98 de l'articulation à baïonnette 36. Cette partie femelle est constituée d'un cylindre creux dont le diamètre intérieur est légèrement supérieur à celui de la partie mâle 60. Deux ergots 100 sont formés à l'intérieur du cylindre 98, disposés et dimensionnés de manière à pouvoir s'insérer et coulisser dans les gorges 64 et 66 de la partie mâle 60 de l'articulation 36.

Les deux coques 70 et 72 sont assemblées par trois vis 102 de manière à assurer le positionnement et le maintien en place des pièces logées dans le boîtier 32. On notera que deux de ces vis traversent les coins 78 de la poche réservoir 74 percés à cet effet.

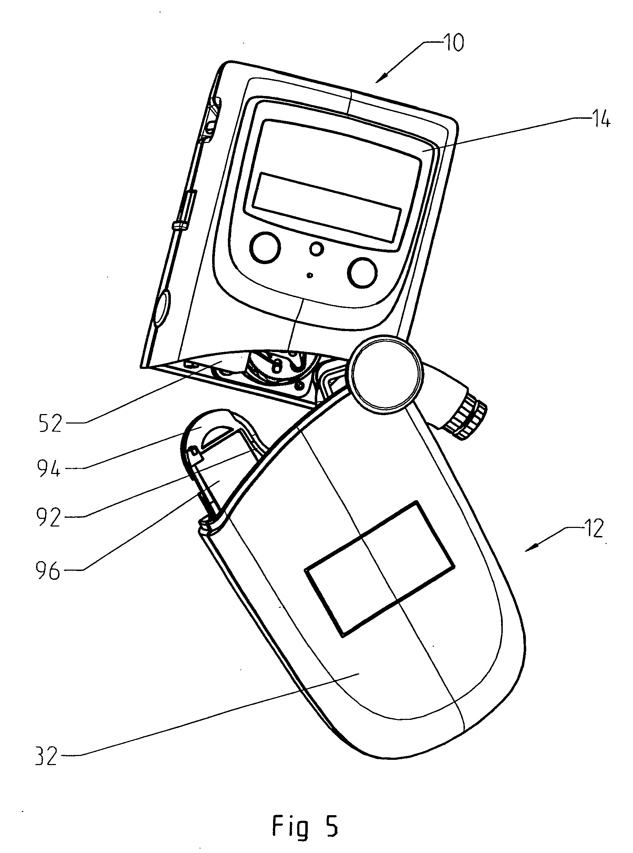

On se référera, pour terminer, à la figure 5 qui montre la façon dont le module pompe 10 et le module cassette 12 sont accouplés à l'aide de l'articulation à baïonnette 36. Du fait du positionnement relatif des deux gorges verticales 66 de la partie mâle 60 et des deux ergots 100 de la partie femelle 98, la partie mâle ne peut pénétrer dans la partie femelle que pour une position angulaire bien déterminée des deux modules l'un par rapport à l'autre. Comme le montre la figure 5, cette position correspond, typiquement, à un angle de 45° entre les faces des deux modules destinées à venir en contact l'un avec l'autre.

Lorsque les deux modules sont ainsi disposés, la partie mâle 60 peut pénétrer à l'intérieur de la partie femelle 98 jusqu'à ce que les ergots 100 arrivent dans la gorge horizontale 64 et butent contre sa paroi. Le pivotement peut ainsi s'effectuer, les ergots 100 se déplaçant horizontalement dans la gorge 64, jusqu'à ce que les deux modules se trouvent accouplés. Il n'est alors plus possible de désengager les deux parties de l'articulation à baïonnette tant que, par un pivotement en sens inverse, les ergots 100 ne sont pas à nouveau alignés avec les gorges verticales 66.

Ainsi est réalisée une liaison amovible et articulée des deux modules qui procure à l'ensemble une grande rigidité qui permet de guider facilement les mouvements relatifs des deux pièces.

Lorsque les modules pompe 10 et cassette 12 sont assemblés et que la pompe fonctionne, la coopération de la rainure et du crochet des parties 26a et 26b du verrou garantit leur accouplement. Néanmoins, il est primordial qu'aucune ouverture intempestive ne puisse se produire. A cet effet, le verrou 26 ne peut être actionné qu'en agissant simultanément sur le bouton 22 et la tirette 24. Les deux mains doivent donc être utilisées pour l'opération, ce qui rend fortement improbable tout désaccouplement involontaire des deux modules.

Le rôle joué par la mémoire du module pompe et la carte mémoire SIM 96 du module cassette va maintenant être exposé.

La mémoire du module pompe 10 sert de support d'enregistrement aux données relatives au patient et à son traitement. ll s'agit, typiquement et principalement, des données suivantes :
- identification du patient ;
- instructions relatives à l'administration de différents médicaments susceptibles d'intervenir dans le traitement : débit d'injection instantané maximum, quantité injectable maximum par jour, nombre de bolus autorisés par jour, volume maximum de chaque bolus, espace minimum entre deux bolus, ... ;
- indication d'éventuelles contre-indications ou intolérances particulières à certains médicaments.

La carte SIM 96 du module cassette 12 sert de support d'enregistrement aux données relatives au patient et au programme d'injection prescrit. Il s'agit, typiquement et principalement, des données suivantes :
- identification du patient ;
- identification du médicament ;
- instructions relatives à l'administration du médicament: programme d'injection (quels débits pendant quelles durées), débit des bolus, leur nombre et leur espacement autorisés.

L'enregistrement des données dans les deux mémoires se fait avantageusement à partir d'un ordinateur selon des techniques bien connues de l'homme de métier.

Dès que les deux modules sont accouplés, le microprocesseur du module pompe 10 vérifie que les données contenues dans la carte SIM 96 sont en accord avec les données de sa propre mémoire. Si tel n'est pas le cas, la pompe ne peut être mise en fonction et l'afficheur 20 fournit un message d'erreur. Si le microprocesseur n'a pas détecté d'anomalie, la pompe est prête à être mise en action selon les instructions fournies par la carte SIM 96.

Il suffit alors que le personnel soignant connecte à la pompe, grâce au raccord 34, un tuyau souple muni d'une aiguille d'injection, purge le circuit en actionnant la vis située derrière le clapet 30, pique le patient et mette la pompe en marche à l'aide du bouton 16.

Le microprocesseur est avantageusement programmé pour interdire la mise en marche de la pompe si la purge n'a pas été effectuée. Par ailleurs, l'accès au septum 84 peut être définitivement obturé dès que, pour la première fois, un médicament a été injecté dans la poche réservoir 74.

Ainsi est proposée une pompe dont la mise en service se fait seulement par des opérations ultra simples ne nécessitant aucune formation spéciale puisque son module cassette, pré-programmé, fournit au module pompe, dès qu'ils sont accouplés, toutes les données relatives au programme d'injection prescrit. Par ailleurs, la présence, dans le module pompe, de données de sécurité relatives au traitement sert à détecter toute anomalie.

Tout risque découlant d'une mauvaise programmation de la pompe au moment de sa mise en service est donc exclu. De plus, l'accouplement mécanique et électrique des deux modules est facilité et procure une grande précision.

## Revendications

1. Pompe programmable pour l'injection de médicaments, comportant deux parties accouplables de manière amovible, soit :
- un module cassette (12) contenant un liquide à injecter et comprenant une mémoire électronique (96) dans laquelle ont été préalablement enregistrées des données relatives au programme d'injection prescrit, et
- un module pompe (10) possédant des moyens d'actionnement qui, lors de l'accouplement, répondent directement aux données contenues dans ladite mémoire (96) pour faire passer le liquide du module cassette (12) vers l'extérieur selon le programme désiré,
**caractérisée en ce que** les modules pompe (10) et cassette (12) sont accouplés au moyen d'une articulation (36).

2. Pompe selon la revendication 1, **caractérisée en ce que** ladite articulation (36) est de type à baïonnette.

3. Pompe selon la revendication 2, **caractérisée en ce que** ladite articulation (36) comporte une partie mâle (60) appartenant à l'un des modules et une partie femelle (98) appartenant à l'autre module.

4. Pompe selon la revendication 3, **caractérisée en ce que** ladite partie mâle (60) est de forme cylindrique et possède, à sa base, une gorge horizontale (64) formée sur tout son pourtour et deux gorges verticales (66) communiquant avec la gorge horizontale.

5. Pompe selon la revendication 4, **caractérisée en ce que** ladite partie femelle (98) est constituée d'un cylindre creux dont le diamètre intérieur est légèrement supérieur à celui de la partie mâle et qui possède deux ergots (100) intérieurs disposés et dimensionnés de manière à pouvoir s'insérer et coulisser dans les gorges (64, 66) de ladite partie mâle.

6. Pompe selon l'une des revendications 1 à 5, **caractérisée en ce qu'**elle comporte un verrou (26) destiné à solidariser le module pompe (10) et le module cassette (12) lorsqu'ils sont accouplés.

7. Pompe selon l'une des revendications 1 à 6, **caractérisée en ce que** le module pompe (10) est de type péristaltique rotatif à galets et **en ce que** le module cassette (12) comporte une poche (74) destinée à contenir le liquide à injecter, un tuyau souple (82) reliant ladite poche à l'extérieur et une contre-pièce (86) sur laquelle lesdits galets viennent tour à tour écraser ledit tuyau (82) et pousser ainsi vers l'extérieur le liquide contenu dans la poche (74).

8. Pompe selon l'une des revendications 1 à 7, **caractérisée en ce que** la mémoire (96) du module cassette (12) est une carte SIM et **en ce que** le module pompe (10) comporte un connecteur (56) relié auxdits moyens d'actionnement et positionné de manière à ce que, lors de l'accouplement des deux modules, ses plages de contact viennent précisément au contact des plages de ladite carte SIM.

9. Pompe selon les revendications 7 et 8, **caractérisée en ce que** la carte SIM (96) est disposée, de manière amovible, sur ladite contre-pièce (86).

10. Pompe selon l'une des revendications 1 à 9, **caractérisée en ce que** le module pompe (10) comporte une mémoire dans laquelle ont été préalablement enregistrées des données de sécurité relatives au traitement.

11. Pompe selon la revendication 10, **caractérisée en ce que** lesdits moyens d'actionnement comportent un microprocesseur programmé pour vérifier la compatibilité des données contenues dans les deux mémoires.

12. Pompe programmable pour l'injection de médicaments, comportant deux parties accouplables de manière amovible, soit :
- un module cassette (12) contenant un liquide à injecter et comprenant une mémoire électronique (96) dans laquelle ont été préalablement enregistrées des données relatives au programme d'injection prescrit, et
- un module pompe (10) possédant des moyens d'actionnement qui, lors de l'accouplement, répondent directement aux données contenues dans ladite mémoire (96) pour faire passer le liquide du module cassette (12) vers l'extérieur selon le programme désiré,
**caractérisée en ce que** le module pompe (10) comporte une mémoire dans laquelle ont été préalablement enregistrées des données de sécurité relatives au traitement et **en ce que** lesdits moyens d'actionnement comportent un microprocesseur programmé pour vérifier la compatibilité des données contenues dans les deux mémoires.
